# EUROPEAN PATENT APPLICATION

(11) **EP 3 718 543 A1**
(43) Date of publication of application: **07.10.2020**
(21) Application number: 17933365.3
(22) Date of filing: 30.11.2017
(51) Int. Cl.: A61K 31/045, A61K 31/343, A61K 31/122, A61K 31/352, A61K 31/015, A61P 31/20, A61P 31/12, A61P 15/02, A61K 36/9066

(54) **PHARMACEUTICAL COMPOSITION AND USE THEREOF**

(71) Applicant: Chen, Rong, Haikou, Hainan 570216 (CN); Xu, Yang, Haikou, Hainan 570216 (CN); Xu, Lang, Haikou, Hainan 570216 (CN)
(72) Inventor: Chen, Rong, Haikou, Hainan 570216 (CN); Xu, Yang, Haikou, Hainan 570216 (CN); Xu, Lang, Haikou, Hainan 570216 (CN)
(74) Representative: Schnappauf, Georg
(86) International application number: PCT/CN2017/113811
(87) International publication number: WO 2019/104587

(57) **Abstract**

A pharmaceutical composition and use thereof. The pharmaceutical composition comprises zedoary turmeric oil and borneol. The zedoary turmeric oil comprises oxysophocarpine (germacrone), curdione, furanodiene, curcumol, elemene and curzerene, and does not comprise 2,4,6-pentamethylaniline, or the content of 2,4,6-pentamethylaniline is less than or equal to 100 ppm. The pharmaceutical composition is used for preventing and/or treating common HPV infection and various diseases caused thereby. The formulation prepared from the pharmaceutical composition has better safety and better clinical effects.

## Description

### FIELD

The present invention belongs to the technical field of medicines, and specifically relates to a pharmaceutical composition and use thereof. The pharmaceutical composition comprises a novel zedoary turmeric oil containing germacrone, curdione, furanodiene, curcumol, β-elemene and curzerene, with no 2,4,6-pentamethylaniline.

### BACKGROUND

Traditional Chinese medicine is a treasure of the Chinese nation and has been playing an important role for accumulated experiences in disease prevention or treatment. With the advancement in traditional Chinese medicine modernization and the implementation of major national special scientific and technical projects, China's traditional Chinese medicine industry has developed rapidly. However, due to the condition limitations during the original research period, most of the traditional Chinese medicines have problems such as broad clinical positioning, undetermined active substances and their mechanism of action, rough pharmaceutical processes, and poor quality control technology, resulting in low quality standards and scientific and technological content of Chinese patent medicines, lacking competitiveness in the international market. Traditional Chinese medicine has been used for 5,000 years, whereas China's new drug approval system has only been established for a few decades. Currently, there are more than 9,000 approved traditional Chinese medicines, with about 58,000 approval numbers, of which only a few have a solid research foundation. Although most of the traditional Chinese medicines are derived from classic prescriptions or proved prescriptions, and have a clinical basis, the preliminary research work therefor that meets the requirements for modern drug research and development is lack, and most ones have not been systematically and canonically researched and their effectiveness and safety have not been evaluated since marketing. Therefore, the secondary development of traditional Chinese medicines is of great significance.

Harald Zur Hausen, a scientist from Germany, was rewarded with Nobel Prize in Physiology or Medicine in 2008 for confirming that persistent HPV infection is necessary for the development of cervical cancer, making it the only preventable or treatable cancer with a clear cause among all existing cancers. Therefore, curing cervical HR-HPV infection can block the progression to cervical cancer. Over the past decade, traditional Chinese medicine has achieved certain success in the treatment of female genital HR-HPV infection, but so far, there is no internationally recognized drug safe and effective for the treatment of HPV infection. Therefore, the development of drugs to treat HR-HPV infection is expected.

The traditional Chinese medicine called "Baofukang suppository" is an external suppository for use in vaginas, functioning to promote qi circulation and eliminate extravasated blood, and promote tissue regeneration and relieve pain. It is mainly used for treatment of leukorrheal diseases cause by dampness and heat, and blood stasis with symptoms such as excess and yellow morbid leukorrhea and sometimes vaginal pruritus; mycotic vaginitis, senile vaginitis, and cervical erosion in patients. Baofukang suppository comprises zedoary turmeric oil and borneol, wherein the zedoary turmeric oil is pungent and warm and can be used to eliminate extravasated blood and activate qi, and remove food retention and relieve pain; and borneol is pungent and cool and can be used to clear away heat and toxic materials, and reduce swelling and relieve pain. They when combined can be used for clearing away heat and activating blood circulation, and reducing swelling and relieving pain. In the past 17 years, basic and clinical researches have shown that "Baofukang suppository" can be used to treat infection and persistent infection, and various diseases caused by HR-HPV, and/or infection and various diseases caused by LR-HPV, and at present, has been the only traditional Chinese medicine with a clinical treatment efficacy in HPV infection confirmed by large-scale RCT clinical research. Therefore, the secondary development of this product is of very important clinical value.

### DETAILED DESCRIPTION

Based on the above reasons, through unremitting research for years, the applicant has developed a novel zedoary turmeric oil, comprising germacrone, curdione, furanodiene, curcumol, β-elemene and curzerene, with no 2,4,6-pentamethylaniline, or the content of 2,4,6-pentamethylaniline is less than or equal to 100 ppm by weight. A pharmaceutical composition comprising the novel zedoary turmeric oil and borneol in combination is used for preventing and/or treating common HPV infection and various diseases caused thereby, including infection or persistent infection and cervical intraepithelial neoplasia CINI, CINII, CINIII, and cervical cancer caused by subtypes of high-risk human papillomavirus (HR-HPV) including but not limited to HPV16, 18, 30, 31, 33, 35, 39, 45, 51, 52, 53, 56, 58, 59, 66, 68, 69; and infection and diseases such as flat wart, papilloma, genital condyloma acuminatum caused by subtypes of low-risk human papillomavirus (LR-HPV) including but not limited to HPV 6, 11, 42, 43, 44, 61, CP8304. In the present invention, a formulation prepared by combining the novel zedoary turmeric oil with borneol has a better safety and a better clinical efficacy.

In the present invention, research on several active ingredients in the zedoary turmeric oil has been performed through several creative experiments, and it is found that, a zedoary turmeric oil with 2,4,6-pentamethylaniline being removed in combination with borneol has a better safety and a better clinical efficacy, laying a material foundation for the secondary development of traditional Chinese medicines.

The present invention is achieved through the following technical solutions:

The prevent invention provides a pharmaceutical composition comprising zedoary turmeric oil and borneol, wherein the zedoary turmeric oil comprises germacrone, curdione, furanodiene, curcumol, β-elemene and curzerene, with no 2,4,6-pentamethylaniline, or the content of 2,4,6-pentamethylaniline is less than or equal to 100 ppm by weight.

In the pharmaceutical composition, the zedoary turmeric oil comprises 5.0 wt%-13.0 wt% of germacrone, 10.0 wt%-30.0 wt% of curdione, 10.0 wt%-26.0 wt% of furanodiene, 0.5 wt%-6.0 wt% of curcumol, 1.0 wt%-6.0 wt% of β-elemene, and 2.0 wt%-21.0 wt% of curzerene, with no 2,4,6-pentamethylaniline, or the content of 2,4,6-pentamethylaniline is less than or equal to 100 ppm by weight.

In the pharmaceutical composition, the zedoary turmeric oil comprises 6.0 wt%-12.0 wt% of germacrone, 13.0 wt%-26.0 wt% of curdione, 12.0 wt%-25.0 wt% of furanodiene, 0.8 wt%-5.0 wt% of curcumol, 2.0 wt%-5.0 wt% of β-elemene, and 7.5 wt%-19.0 wt% of curzerene, with no 2,4,6-pentamethylaniline, or the content of 2,4,6-pentamethylaniline is less than or equal to 10 ppm by weight.

In the pharmaceutical composition, the zedoary turmeric oil comprises 6.0 wt%-12.0 wt% of germacrone, 13.0 wt%-26.0 wt% of curdione, 12.0 wt%-25.0 wt% of furanodiene, 0.8 wt%-5.0 wt% of curcumol, 2.3 wt%-4.0 wt% of β-elemene, and 8 wt%-16 wt% of curzerene, with no 2,4,6-pentamethylaniline.

The prevent invention provides use of the pharmaceutical composition in the manufacture of a medicament for prevention and/or treatment of HPV infection, including infection or persistent infection as well as CINI, CINII, CINIII, and cervical cancer caused by various subtypes of high-risk human papillomavirus (HR-HPV); and/or infection and genital condyloma acuminatum caused by various subtypes of low-risk human papillomavirus (LR-HPV);
wherein the HR-HPV comprises HPV 16, 18, 30, 31, 33, 35, 39, 45, 51, 52, 53, 56, 58, 59, 66, 68, and 69; and the LR-HPV comprises HPV 6, 11, 42, 43, 44, 61 and CP8304.

The present invention provides the pharmaceutical composition formulated as a pharmaceutical preparation.

The pharmaceutical preparation is in a dosage form of an external preparation, an oral preparation, or an injection preparation.

The weight ratio of the zedoary turmeric oil to borneol in the pharmaceutical composition is 164: 150.

The prevent invention provides use of the pharmaceutical composition in a medicament for prevention and/or treatment of infection by various viruses, bacteria, fungi, chlamydiae, mycoplasmas, trichomonas; infection of eye, ear, nose and throat; vulvitis, various vaginitis, mixed infectious vaginitis, skin infection, mucosal infection, hemorrhoid infection, constipation, cervicitis, adnexitis, anti-oxidation.

The external preparation comprises the zedoary turmeric oil, borneol, and a transdermal absorption enhancer.

The transdermal absorption enhancer is one or more selected from laurocapram, dimethyl sulfoxide, tetradecanol, caprylic acid and oleic acid.

The zedoary turmeric oil described in the present invention may be prepared by the following method:
The extracted zedoary turmeric oil is obtained by an SFE-CO₂ method with 95% ethanol as an entrainer. The preparation method comprises, but is not limited to: taking fresh zedoray rhizome from a cold storage that has been sterilized entirely including all containers and spaces, finely washing, cutting into slices of 2-3 mm, drying at 40-60°C, pulverizing into fine powders of zedoray rhizome, then passing through a 40-80-mesh sieve, putting the fine powders of zedoray rhizome into an extraction kettle, which is adjusted to a working pressure of 10-12 MPa and a working temperature of 40-50°C; and performing extraction for 90-100 minutes to obtained the extracted zedoary turmeric oil, wherein the first-stage separation column is at a working pressure of 7-9 MPa and a working temperature of 50-60°C; and the second-stage separation column is at a working pressure of 4-5 MPa and a working temperature of 30-40°C; adding ethyl acetate or dichloromethane into the extracted zedoary turmeric oil till it is dissolved completely, then adding a 0.01 mol/L hydrochloric acid aqueous solution, stirring, allowing for standing, retaining the ethyl acetate layer or the dichloromethane layer, and then recovering the organic solvent completely, to obtain a novel zedoary turmeric oil.

The zedoary turmeric oil of the present invention may be prepared according to the following method:
The commercially available steam-distilled zedoary turmeric oil is purchased from Shenzhen Guoxin Flavor & Fragrance Co, Ltd. on January 8, 2016.

Ethyl acetate or dichloromethane is added into the commercially available steam-distilled zedoary turmeric oil till it is dissolved completely, then a 0.01 mol/L hydrochloric acid aqueous solution is added, stirred, and allowed for standing, and the ethyl acetate layer or the dichloromethane layer is retained, and then the organic solvent is recovered completely to obtain a novel zedoary turmeric oil.

The following is described in the present invention: germacrone, CAS No. 6902-91-6; curdione, CAS No. 13657-68-6; furanodiene, CAS No. 19912-61-9; curcumol, CAS No. 4871-97-0; curzerene, CAS No. 17910-09-7; and β-elemene, CAS No. 515-13-9.

### Detection and analysis for the zedoary tumeric oil:

Chromatographic condition and system suitability: an octadecylsilane-bonded silica gel is used as a filler; gradient elution is performed as specified in the table below with acetonitrile as mobile phase A, and water as mobile phase B; detection is performed at a wavelength of 216 nm. The theoretical plate number is not less than 5000 calculated based on the peak of germacrone.

**Table 1. Gradient mobile phase**

| Time (minutes) | Mobile Phase A (%) | Mobile Phase B (%) |
|---|---|---|
| 0-15 | 75 | 25 |
| 16-25 | 75→790 | 25→710 |
| 25-35 | 90 | 10 |

Preparation of a reference solution: an appropriate amount of a germacrone control and a furanodiene control are weighted precisely, and absolute ethanol is added to obtain a mixed solution, containing, per 1 ml, 30 µg of germacrone, 50 µg of furanodiene, 35 µg of curdione, 50 µg of curcumol, 30 µg of curzerene, and 30 µg of β-elemene.

Preparation of a tested solution: 0.1 g of the present product is weighed precisely, and put in a 50 ml volumetric flask, and absolute ethanol is added up to the mark, and shaked homogeneously. 5 ml of the mixture is precisely measured, and then put in a 25 ml volumetric flask, absolute ethanol is added up to the mark, shaked homogeneously, and filtered, and the subsequent filtrate is collected.

Detection: 5 µl of each of the reference solution and the tested solution is precisely drawn and injected into a liquid chromatograph, and detected, and the chromatogram is recorded.

Detection of content of 2,4,6-pentamethylaniline: it is performed by Hainan Institute For Drug Control.

### Examples

The technical solutions of the present invention are described below with reference to specific examples, but the protection scope of the present invention is not limited thereto.

The content described in the Examples of the present specification merely illustrates the implementation forms of the inventive concept. The protection scope of the present invention should not be regarded as limited merely to the specific forms stated in the Examples. The protection scope of the present invention is also based on the equivalent technical means conceivable for those skilled in the art in light of the inventive concept. Although the embodiments of the present invention are described below, the present invention is not limited to the specific embodiments and application fields described above, and the specific embodiments described below are merely illustrative and instructive, and not limiting. Under the inspiration of the specification and without departing from the protection scope of the claims of the present invention, those of ordinary skill in the art could provide more embodiments, all of which fall within the protection scope of the present invention.

The following tests of the present invention are conclusive ones summarized by the researchers by many creative tests based on the technical solutions claimed by the present invention.

### Test 1

### Process study

In the present invention, extracted zedoary turmeric oil was obtained by an SFE-CO₂ method with 95% ethanol as an entrainer. Fresh zedoray rhizome was taken from a cold storage that had been sterilized entirely including all containers and spaces, finely washed, cut into slices of 2-3 mm, dried at 50°C, pulverized into fine powders of zedoray rhizome, which then were passed through a 60-mesh sieve. Fine powders of zedoray rhizome were put into an extraction kettle, which was adjusted to a working pressure of 11 MPa and a working temperature of 45°C. Extraction was performed for 95 minutes to obtain the extracted zedoary turmeric oil, wherein the first-stage separation column was at a working pressure of 8 MPa and a working temperature of 55°C; and the second-stage separation column was at a working pressure of 4.5 MPa and a working temperature of 35°C. Ethyl acetate was added into the zedoary turmeric oil till it was dissolved completely, then a 0.01 mol/L hydrochloric acid aqueous solution was added, stirred, and allowed for standing. The ethyl acetate layer was retained, and then the organic solvent was recovered completely, to obtain a novel zedoary turmeric oil.

Comparative method: the zedoary turmeric oil was obtained by steam distillation.

Test results were shown in Table 2 below.

**Table 2. Comparison of active ingredients in the "zedoary turmeric oil"**

| Components | Relative content | | |
|---|---|---|---|
| | The present method (SFE) | SD | SFE/SD |
| Furanodiene | 19.20% | 18.31% | 1.05 |
| Germacrone | 11.92% | 11.02% | 1.44 |
| Curdione | 23.78% | 19.96% | 1.19 |
| Curcumol | 4.12% | 3.56% | 1.16 |
| Curzerene | 4.20% | 18% | 0.23 |
| β-elemene | 3.80% | 2.4% | 1.58 |
| 2,4,6-pentamethylaniline | Not detected | 0.23% | - |

Unexpectedly, as compared to the SD method, the irritant ingredients in the zedoary turmeric oil obtained by the present method are reduced by 11, and the ratio thereof is reduced from 17.43% to 1.31% with a decrease of 16.12%; and meanwhile, the main effective ingredients are increased. Therefore, the zedoary turmeric oil of the present invention not only has a greatly reduced irritation, and but also an increased efficacy.

**Table 3. Comparison of irritant chemical ingredients in the "zedoary turmeric oil" obtained by different methods**

| No. | Components | Relative content (%) | | | Irritation or Toxicity |
|---|---|---|---|---|---|
| | | SFE | SD | SFE/SD | |
| 1 | (1R)-(+)-α-pinene | 0.01 | 0.55 | 0.018 | Serious eye irritation, skin irritation, and allergic skin reaction. |
| 2 | Camphene | 0.02 | 0.76 | 0.026 | Irritation to eyes, nose, and throat. |
| 3 | Sabinene | - | 0.15 | 0 | Irritation under local use or oral administration. |
| 4 | β-pinene | 0.01 | 1.01 | 0.0099 | Strong irritation. |
| 5 | D-camphor | 0.02 | 2.40 | 0.0083 | Irritation to nose. |
| 6 | Eucalyptol | 0.16 | 9.44 | 0.017 | Risk of serious eye damage. Irritation to respiratory tract and skin. |
| 7 | Caryophyllene oxide | 0.05 | 0.11 | 0.45 | Irritation to eyes and skin. |
| 8 | 2-heptanone | - | 0.03 | 0 | Strong irritation to nose and eyes. |
| 9 | 2-heptanol | - | 0.06 | 0 | Irritation to eyes and skin. |
| 10 | Myrcene | - | 0.18 | 0 | Irritation to eyes, respiratory system and skin. |
| 11 | α-terpinene | - | 0.04 | 0 | Irritation. |
| 12 | m-isopropyltoluene | - | 0.04 | 0 | Moderate toxicity. |
| 13 | γ-terpinene | - | 0.08 | 0 | Irritation. |
| 14 | Terpinolene | - | 0.04 | 0 | Irritation to eyes, skin and upper respiratory tract. |
| 15 | Linalool | - | 0.95 | 0 | Induction of low spirits, depression, or even life-threatening respiratory diseases. |
| 16 | Benzofuran | - | 0.12 | 0 | Poisoning after inhalation, ingestion, or transdermal absorption. Irritation. |
| 17 | 2-nonanone | - | 0.04 | 0 | Irritation to eyes and skin. |
| 18 | γ-eudesmol | 0.18 | 0.28 | 0.64 | Irritation to eyes and skin. |
| 19 | Cyclohexane | 0.13 | 0.28 | 0.46 | Toxic, and irritation to eyes and skin. |
| 20 | β-eudesmol | 0.73 | 0.87 | 0.84 | Irritation to eyes and skin. |
| Total | | 1.31 | 17.43 | 0.075 | |

| | | | | | |
|---|---|---|---|---|---|
| Note: "-" in the table means not detected. | | | | | |

The four main active ingredients retained in the pharmaceutical composition of the present invention not only have a high content, but also have well-established pharmacological activities, which are briefly described as follows:
1. Furanodiene: Furanodiene shows an inhibitory effect on tumor cells in both *in vivo* and *in vitro* experiments. Under furanodiene, human promyelocytic leukemia HL-60, gastric cancer SGC-7901, and prostate cancer PC3 cells are strongly inhibited and killed. By Liang Haiyan using a CCK-8 method, furanodiene is determined to inhibit the proliferation of cervical cancer cells, SiHa and HeLa.
2. Germacrone: Germacrone shows an inhibitory effect on tumors including human ovarian cancer A2780, colon cancer HCT and nasopharyngeal carcinoma KB3, resulting in inhibiting the proliferation of human ovarian cancer A2780 cells, colon cancer HCT cells and nasopharyngeal carcinoma KB3 cells. Under germacrone, the viral antigen expression and viral genome replication is inhibited in a dose-dependent way, and amantadine-resistant strains, influenza B virus and influenza A virus H3N2 are resisted.
3. Curdione: Curdione shows an obvious inhibitory effect on sarcoma 37, cervical cancer U14 and Ehrlich's ascites carcinoma in mice, resulting in necrocytosis of cancer cells. Mice with Ehrlich' ascites carcinoma treated with the present product can successfully gain active immunity. Clinical results show that curdione display a better efficacy in the treatment of cervical cancer. Curdione further displays anti-inflammatory and analgesic effects.
4. Curcumol: Curcumol is a component indicative of zedoary turmeric oil under many versions of Chinese Pharmacopoeia and can be used to enhance the activity of immune cells as well as the body's immunity and immunogenicity. For sarcoma S37 in mice, the inhibition rate at a dose of 75 mg/kg is 53.47-61.96%; and the inhibition rate for cervical cancer U14 in mice at a dose of 75 mg/kg is 45.1-77.13%. Clinical reports indicate that curcumol displays a better efficacy in the treatment of cervical cancer. For Ehrlich's ascites carcinoma (EAC) in mice, the inhibition rate is 65.8-78.9% at a dose of 75 mg/kg. Curcumol results in obviously inhibiting the growth of tumors in H22 tumor-bearing mice; inducing apoptosis of HT-29 and HepG2 cells *in vitro;* inhibiting the proliferation of human colon cancer SW1116 and inducing apoptosis thereof; and further displays a stronger antifungal activity, with no obvious adverse reactions.

### Test 2

### Study on acute toxicity

Test Group 1: 118 g of zedoary turmeric oil (the zedoary turmeric oil of the present invention in Test 1), and 108 g of borneol;
Preparation process: 118 g of the zedoary turmeric oil of the present invention and 108 g of borneol were added to an appropriate amount of ethanol, stirred, and dissolved. Additionally, 49.00 g of polyethylene glycol 4000 was taken, heated, and melt, 2.78 g of laurocapram was added and mixed homogeneously, then the above drug solution was added, mixed homogeneously, poured into a suppository mold, and removed after cooling, to obtain 1000 suppositories.

Test process: Rats in the test group 1 were administered 2 suppositories of the tested drugs, twice a day at an interval of at least 4 hours, for 1 day. Observation was continuously performed for at least 4 hours after administration, and thereafter once a day for 14 days.

Test results: When the dose had reached 2419 ± 112 mg/kg, there were no obvious abnormalities in animals. No animals died after administration until the end of the observation period in the test. All animals were necropsied for general examination on d15 after administration. No abnormal secretions were found in the physiological cavity and tract of all animals. Main organs in the thoracic and abdominal cavities including the trachea, esophagus, thymus, adrenal gland, pancreas, stomach, intestine (duodenum, jejunum, ileum, colon, rectum), heart, lung, liver, spleen, kidney, brain, lymph nodes, bladder, uterus, ovary, and vagina (the administration site) showed normal anatomical positions, a glossy and normal color, and a uniform texture, and was not abnormally increased or decreased. No obvious toxicity was observed in an acute toxicity test, wherein SD rats were dosed vaginally at an equivalent dose 886 ± 41 times larger than a human clinical daily dose based on body weight in kilograms.

Test conclusions: The maximum tolerated dose of the traditional Baofukang suppository was 786.7 ± 32 mg/kg, whereas the maximum tolerated dose of the preparation prepared from the novel zedoary turmeric oil of the present invention was greater than 2419 ± 112 mg/kg, which had a wider safety window, further illustrating that the preparation of the present invention had a better safety. The Zedoary turmeric oil with 2,4,6-pentamethylaniline removed (or the content of which was very low) when combined with borneol, showed a significantly increased maximum tolerated dose, and a wider safety window.

### Test 3

### Special safety test study

### (1) Allergy test

The tested drug was the same as that used in the test group 1 in Test 2 The guinea pigs were vaginally administered once every 7 days for sensitization. Sensitization was performed continuously 3 times, and 14d after the last sensitization, each guinea pig was dosed with 0.1 g for challenge. There were no abnormal symptoms such as edema, hyperemia and secretions observed in the vulva of the animals in the tested drug group after 24 hours and 48 hours. The vagina of guinea pigs was dissected 48 hours after challenge and examined visually, and the vaginal mucosa of the animals in the tested drug group did not show hyperemia, edema and other symptoms of vaginal local allergic reactions.

This indicated that, the vaginal mucosa of rabbits was not sensitized by the tested drug.

### (2) Irritation test

The tested drug was the same as that used in the test group 1 in Test 2. The tested drugs were given in the intact vagina group and the damaged vagina group regularly every day by slowly pushing 2 suppositories deep inside the vagina with a sterile glass cannula. Before administration, rabbits were stimulated to urinate with a sterilized glass cannula. After administration, rabbits were fixed in a supine position for 10 min and then returned. The administration was performed continuously for 7 days. The female rabbits were sacrificed and dissected within 24 hours after the last administration, and the vagina was visually examined and recorded for congestion and inflammation. The vaginal tissues were taken, fixed with a 10% formaldehyde solution, divided into three sections of upper, middle and lower ones, and prepared into 5 µm paraffin-embedded sections. The conventional staining was used to judge the degree of histological inflammation stimulation and observation was performed under light microscope. According to the Eckstein criteria, scoring was based on four indexes: hyperemia, edema, inflammatory cell infiltration and epithelial cell necrosis.

Test conclusions: The irritation of the pharmaceutical composition of the present invention was scored 1.3 in total, which indicated that the tested drug displayed no obvious irritation on both the rabbits' complete vagina and damaged vagina, suggesting that the vaginal irritation test results of the tested product had met the regulations on local medication.

### Test 4

Test group 1: 82 g of zedoary turmeric oil (the zedoary turmeric oil of the present invention in the test group 1), and 75 g of borneol;

Preparation process: 82 g of zedoary turmeric oil and 75 g of borneol were added into an appropriate amount of ethanol, stirred and dissolved. Additionally, 1235 g of polyoxyl (40) stearate and 200 g of polyethylene glycol 4000 were taken, heated and melt. 120 g of polyethylene glycol 400 and 17.5 g of laurocapram were added and mixed homogeneously, then the above drug solution was added, mixed homogeneously, then poured into a suppository mold, and removed after cooling to obtain 1000 suppositories.

### Therapeutic effect on HR-HPV infection

Participants: This study was conducted at the Affiliated Hospital of Hainan Medical University. From February 2016 to February 2017, 42 patients averagely aged 34.16 ± 6.57 years were definitely diagnosed with cervical HR-HPV infection in the gynecological outpatient department and all had signed informed consent.

Inclusion criteria: (1) women ≥18 years with a history of coitus; (2) positive for HR-HPV DNA detection; (3) no abnormal epithelial cells and cancer cells observed in the cervical cytology detection; (4) no major organ diseases; (5) no history of drug allergies (especially for those with allergic hereditary).

Treatment process: 1 to 2 days after menstruation, the patients were administrated 2 suppositories in the test 4 every night for 16 days in a month, with 3 menstrual cycles as a course of treatment. All patients received full treatment and follow-up observation as required, and used contraception. The first visit was performed in the fourth month after 3 months of treatment. The negative status of HR-HPV DNA in patients was detected, and the patient was cured if the subtype was turned to be negative.

Test results: see table 4.

**Table 4. Negative rate for HR-HPV infection**

| HR-HPV | Negative cases | All cases | Negative rate |
|---|---|---|---|
| 16 | 10 | 13 | 76.93% |
| 18 | 3 | 5 | 60.00% |
| 33 | 1 | 2 | 50.00% |
| 52 | 7 | 9 | 77.78% |
| 56 | 2 | 4 | 50.00% |
| 58 | 2 | 4 | 50.00% |
| 68 | 5 | 5 | 100.00% |
| Total | 30 | 42 | 71.43% |

Test conclusions: The pharmaceutical composition prepared from the novel zedoary turmeric oil of the present invention and borneol displays a good effect in turning HPV infection to be negative.

### Test 5

The therapeutic effect of the suppository prepared from the zedoary turmeric oil of the present invention and borneol on CIN I, CIN II, CIN III:

Research subjects: This study was conducted at the Shenyang Women's and Children's Hospital. From January 2016 to January 2017, 116 patients were diagnosed histologically with CIN I, CIN II, or CIN III, and aged from 18 to 42 years, with an average age of 36.77 ± 8.20 years. Informed consent was signed voluntarily.

Test process: 116 patients were firstly examined by pathological immunohistochemical detection for the expression of Ki67, P16 and P53 proteins, wherein P16 was a novel antioncogene; P53 was an important tumor suppressor gene; and Ki67 expression was closely related to cell proliferation activity.

38 cases for each of CIN I and CIN II were treated without surgery, and dosed 1-2 days after menstruation; and 40 cases for CIN III were subjected to Leep or cold knife conization, and dosed from d7 after surgery. Vulva was cleaned before sleep, and the pharmaceutical composition in the test 4 was inserted into deep inside the vagina with a dose of 2 suppositories, once a night, for 16 days/month for 6 months. Cervical tissues were taken and biopsied under colposcopy 3 to 7 days after administration had been stopped, and pathological immunohistochemistry was used to determine the expression of Ki67, P16 and P53 proteins again, which was compared with protein expression before drug administration.

The test results were shown in Table 5-Table 7.

**Table 5. Expression of P16 protein in CIN patients before and after treatment with the suppositories of the pharmaceutical composition of the present invention**

| Group | Cases | Before | | After | | χ² | P-Value |
|---|---|---|---|---|---|---|---|
| | | Positive cases | Positive (%) | Positive cases | Positive (%) | | |
| CINI | 38 | 11 | 28.95 | 3 | 7.89 | 5.345 | 0.018 |
| CINII | 38 | 18 | 47.37 | 5 | 13.16 | 10.537 | 0.001 |
| CINIII | 40 | 23 | 57.50 | 11 | 27.50 | 7.366 | 0.007 |

**Table 6. Expression of p53 protein in CIN patients before and after treatment with the suppositories of the pharmaceutical composition of the present invention**

| Group | Cases | Before | | After | | χ² | P value |
|---|---|---|---|---|---|---|---|
| | | Positive cases | Positive (%) | Positive cases | Positive (%) | | |
| CINI | 38 | 9 | 23.68 | 2 | 5.26 | 5.208 | 0.022 |
| CINII | 38 | 16 | 42.10 | 5 | 13.16 | 7.962 | 0.005 |
| CINIII | 40 | 21 | 52.50 | 9 | 22.50 | 7.680 | 0.006 |

**Table 7. Expression of ki67 protein in CIN patients before and after treatment with the suppositories of the pharmaceutical composition of the present invention**

| Group | Cases | Before | | After | | χ² | P value |
|---|---|---|---|---|---|---|---|
| | | Positive cases | Positive (%) | Positive cases | Positive (%) | | |
| CINI | 38 | 7 | 18.42 | 1 | 2.63 | 5.029 | 0.025 |
| CINII | 38 | 15 | 39.47 | 4 | 10.53 | 8.491 | 0.004 |
| CINIII | 40 | 19 | 47.50 | 8 | 20.00 | 6.765 | 0.009 |

Conclusions: By treatment of CIN with the pharmaceutical composition of the present invention, the expression of ki67, P16 and P53 protein were reduced, suggesting that the present pharmaceutical composition displayed a good efficacy in the treatment of CIN I, CIN II, and CIN III, thereby inhibiting the activity of tumor cells and preventing the occurrence of cervical cancer.

### Preparation Examples

### Example 1

Preparation of the present zedoary turmeric oil: the extracted zedoary turmeric oil was obtained by an SFE-CO₂ method with 95% ethanol as an entrainer. Fresh zedoray rhizome was taken from a cold storage that had been sterilized entirely including all containers and spaces, finely washed, cut into slices of 2-3 mm, dried at 40°C, pulverized into fine powders of zedoray rhizome, which then were passed through a 40-mesh sieve. Fine powders of zedoray rhizome were put into an extraction kettle, which was adjusted to a working pressure of 10 MPa and a working temperature of 40°C. Extraction was performed for 90 minutes to obtain the extracted zedoary turmeric oil, wherein the first-stage separation column was at a working pressure of 7 MPa and a working temperature of 50°C; and the second-stage separation column was at a working pressure of 4 MPa and a working temperature of 30°C. Ethyl acetate was added into the zedoary turmeric oil till it was dissolved completely, then a 0.01 mol/L hydrochloric acid aqueous solution was added, stirred, and allowed for standing. The ethyl acetate layer was retained, and then the organic solvent was recovered completely, to obtain a novel zedoary turmeric oil.

The novel zedoary turmeric oil comprised 6.13 wt% of germacrone, 16.24 wt% of curdione, 12.10 wt% of furanodiene, 3.85 wt% of curcumol, 2.15 wt% of β-elemene, and 14.10 wt% of curzerene, and the content of 2,4,6-pentamethylaniline is 8 ppm by weight.

Preparation prescription: 164 g of the zedoary turmeric oil of the present invention and 150 g of borneol;
Preparation process: 164 g of zedoary turmeric oil and 150 g of borneol were added into an appropriate amount of ethanol, stirred, and dissolved. Additionally, 1235 g of polyoxyl (40) stearate and 125 g of polyethylene glycol 4000 were taken, heated, and melt. 38 g of polyethylene glycol 400 and 17.5 g of laurocapram were added and mixed homogeneously, then the above drug solution was added and mixed homogeneously, then poured into a suppository mold, and removed after cooling to obtain 1000 suppositories.

### Example 2

The extracted zedoary turmeric oil was obtained by an SFE-CO₂ method with 95% ethanol as an entrainer. Fresh zedoray rhizome was taken from a cold storage that had been sterilized entirely including all containers and spaces, finely washed, cut into slices of 2-3 mm, dried at 50°C, pulverized into fine powders of zedoray rhizome, which then were passed through a 60-mesh sieve. Fine powders of zedoray rhizome were put into an extraction kettle, which was adjusted to a working pressure of 11 MPa and a working temperature of 45°C. Extraction was performed for 95 minutes to obtain the extracted zedoary turmeric oil, wherein the first-stage separation column was at a working pressure of 8 MPa and a working temperature of 55°C; and the second-stage separation column was at a working pressure of 4.5 MPa and a working temperature of 45°C. Ethyl acetate was added into the zedoary turmeric oil till it was dissolved completely, then a 0.01 mol/L hydrochloric acid aqueous solution was added, stirred, and allowed for standing. The ethyl acetate layer was retained, and then the organic solvent was recovered completely, to obtain a novel zedoary turmeric oil.

The novel zedoary turmeric oil comprised 7.85 wt% of germacrone, 25.81 wt% of curdione, 25.00 wt% of furanodiene, 4.80 wt% of curcumol, 3.41 wt% of β-elemene, and 10.02 wt% of curzerene, with no 2,4,6-pentamethylaniline .

Preparation prescription: 164 g of the zedoary turmeric oil of the present invention and 150 g of borneol;
Preparation process: 164 g of zedoary turmeric oil and 150 g of borneol were added into an appropriate amount of ethanol, stirred, and dissolved. Additionally, 1235 g of polyoxyl (40) stearate and 125 g of polyethylene glycol 4000 were taken, heated, and melt. 38 g of polyethylene glycol 400 and 17.5 g of laurocapram were added and mixed homogeneously, then the above drug solution was added and mixed homogeneously, then poured into a suppository mold, and removed after cooling to obtain 1000 suppositories.

### Example 3

The extracted zedoary turmeric oil was obtained by an SFE-CO₂ method with 95% ethanol as an entrainer. Fresh zedoray rhizome was taken from a cold storage that had been sterilized entirely including all containers and spaces, finely washed, cut into slices of 2-3 mm, dried at 50°C, pulverized into fine powders of zedoray rhizome, which then were passed through a 60-mesh sieve. Fine powders of zedoray rhizome were put into an extraction kettle, which was adjusted to a working pressure of 11 MPa and a working temperature of 45°C. Extraction was performed for 95 minutes to obtain the extracted zedoary turmeric oil, wherein the first-stage separation column was at a working pressure of 8 MPa and a working temperature of 55°C; and the second-stage separation column was at a working pressure of 4.5 MPa and a working temperature of 35°C. Ethyl acetate was added into the zedoary turmeric oil till it was dissolved completely, then a 0.01 mol/L hydrochloric acid aqueous solution was added, stirred, and allowed for standing. The ethyl acetate layer was retained, and then the organic solvent was recovered completely, to obtain a novel zedoary turmeric oil.

The novel zedoary turmeric oil comprised 11.92 wt% of germacrone, 23.78 wt% of curdione,19.20 wt% of furanodiene, 4.12 wt% of curcumol, 3.80 wt% of β-elemene, and 4.20 wt% of curzerene, with no 2,4,6-pentamethylaniline .

Preparation prescription: 164 g of the zedoary turmeric oil of the present invention and 150 g of borneol;
Preparation process: 164 g of zedoary turmeric oil and 150 g of borneol were added into an appropriate amount of ethanol, stirred, and dissolved. Additionally, 1235 g of polyoxyl (40) stearate and 125 g of polyethylene glycol 4000 were taken, heated, and melt. 38 g of polyethylene glycol 400 and 17.5 g of laurocapram were added and mixed homogeneously, then the above drug solution was added and mixed homogeneously, then poured into a suppository mold, and removed after cooling to obtain 1000 suppositories.

### Example 4

The extracted zedoary turmeric oil was obtained by an SFE-CO₂ method with 95% ethanol as an entrainer. Fresh zedoray rhizome was taken from a cold storage that had been sterilized entirely including all containers and spaces, finely washed, cut into slices of 2-3 mm, dried at 40°C, pulverized into fine powders of zedoray rhizome, which then were passed through a 40-mesh sieve. Fine powders of zedoray rhizome were put into an extraction kettle, which was adjusted to a working pressure of 10 MPa and a working temperature of 40°C. Extraction was performed for 90 minutes to obtain the extracted zedoary turmeric oil, wherein the first-stage separation column was at a working pressure of 7 MPa and a working temperature of 50°C; and the second-stage separation column was at a working pressure of 4 MPa and a working temperature of 40°C. Dichloromethane was added into the zedoary turmeric oil till it was dissolved completely, then a 0.01 mol/L hydrochloric acid aqueous solution was added, stirred, and allowed for standing. The dichloromethane layer was retained, and then the organic solvent was recovered completely, to obtain a novel zedoary turmeric oil.

The novel zedoary turmeric oil comprised 8.41 wt% of germacrone, 23.18 wt% of curdione, 17.90 wt% of furanodiene, 3.11 wt% of curcumol, 3.05 wt% of β-elemene, and 11.05 wt% of curzerene, with no 2,4,6-pentamethylaniline .

Preparation prescription: 164 g of the zedoary turmeric oil of the present invention and 150 g of borneol;
Preparation process: 164 g of zedoary turmeric oil and 150 g of borneol were added into an appropriate amount of ethanol, stirred, and dissolved. Additionally, 1235 g of polyoxyl (40) stearate and 125 g of polyethylene glycol 4000 were taken, heated, and melt. 38 g of polyethylene glycol 400 and 17.5 g of laurocapram were added and mixed homogeneously, then the above drug solution was added and mixed homogeneously, then poured into a suppository mold, and removed after cooling to obtain 1000 suppositories.

### Example 5

The extracted zedoary turmeric oil was obtained by an SFE-CO₂ method with 95% ethanol as an entrainer. Fresh zedoray rhizome was taken from a cold storage that had been sterilized entirely including all containers and spaces, finely washed, cut into slices of 2-3 mm, dried at 60°C, pulverized into fine powders of zedoray rhizome, which then were passed through a 80-mesh sieve. Fine powders of zedoray rhizome were put into an extraction kettle, which was adjusted to a working pressure of 12 MPa and a working temperature of 50°C. Extraction was performed for 100 minutes to obtain the extracted zedoary turmeric oil, wherein the first-stage separation column was at a working pressure of 9 MPa and a working temperature of 60°C; and the second-stage separation column was at a working pressure of 5 MPa and a working temperature of 40°C. Dichloromethane was added into the zedoary turmeric oil till it was dissolved completely, then a 0.01 mol/L hydrochloric acid aqueous solution was added, stirred, and allowed for standing. The dichloromethane layer was retained, and then the organic solvent was recovered completely, to obtain a novel zedoary turmeric oil.

The novel zedoary turmeric oil comprised 7.89 wt% of germacrone, 21.78 wt% of curdione, 18.34 wt% of furanodiene, 3.04 wt% of curcumol, 2.91 wt% of β-elemene, and 10.83 wt% of curzerene, with no 2,4,6-pentamethylaniline .

Preparation prescription: 164 g of the zedoary turmeric oil of the present invention and 150 g of borneol;
Preparation process: 164 g of zedoary turmeric oil and 150 g of borneol were added into an appropriate amount of ethanol, stirred, and dissolved. Additionally, 1235 g of polyoxyl (40) stearate and 125 g of polyethylene glycol 4000 were taken, heated, and melt. 38 g of polyethylene glycol 400 and 17.5 g of laurocapram were added and mixed homogeneously, then the above drug solution was added and mixed homogeneously, then poured into a suppository mold, and removed after cooling to obtain 1000 suppositories.

### Example 6

The extracted zedoary turmeric oil was obtained by an SFE-CO₂ method with 95% ethanol as an entrainer. Fresh zedoray rhizome was taken from a cold storage that had been sterilized entirely including all containers and spaces, finely washed, cut into slices of 2-3 mm, dried at 50°C, pulverized into fine powders of zedoray rhizome, which then were passed through a 60-mesh sieve. Fine powders of zedoray rhizome were put into an extraction kettle, which was adjusted to a working pressure of 11 MPa and a working temperature of 45°C. Extraction was performed for 95 minutes to obtain the extracted zedoary turmeric oil, wherein the first-stage separation column was at a working pressure of 8 MPa and a working temperature of 55°C; and the second-stage separation column was at a working pressure of 4 MPa and a working temperature of 45°C. Dichloromethane was added into the zedoary turmeric oil till it was dissolved completely, then a 0.01 mol/L hydrochloric acid aqueous solution was added, stirred, and allowed for standing. The dichloromethane layer was retained, and then the organic solvent was recovered completely, to obtain a novel zedoary turmeric oil.

The novel zedoary turmeric oil comprised 8.24 wt% of germacrone, 22.46 wt% of curdione, 18.05 wt% of furanodiene, 3.11 wt% of curcumol, 2.75 wt% of β-elemene, and 11.28 wt% of curzerene, with no 2,4,6-pentamethylaniline .

Preparation prescription: 164 g of the zedoary turmeric oil of the present invention and 150 g of borneol;
Preparation process: 164 g of zedoary turmeric oil and 150 g of borneol were added into an appropriate amount of ethanol, stirred, and dissolved. Additionally, 1235 g of polyoxyl (40) stearate and 125 g of polyethylene glycol 4000 were taken, heated, and melt. 38 g of polyethylene glycol 400 and 17.5 g of laurocapram were added and mixed homogeneously, then the above drug solution was added and mixed homogeneously, then poured into a suppository mold, and removed after cooling to obtain 1000 suppositories.

The content of the novel zedoary turmeric oil from different batches obtained by the present method was shown in Table 8:

**Table 8. The content of active ingredients in the novel zedoary turmeric oil from different batches**

| Batch No. | Germacrone | Curdione | Furanodiene | Curcumol | β-elemene | Curzerene | 2,4,6-penta methylanili ne |
|---|---|---|---|---|---|---|---|
| 20160311 | 5.02% | 30.00% | 12.01% | 0.84% | 2.30% | 2.02% | 9.98 ppm |
| 20160721 | 6.02% | 27.01% | 25.00% | 5.03% | 1.04% | 15.96% | No |
| 20160804 | 11.98% | 26.02% | 10.00% | 0.50% | 5.99% | 21.01% | No |
| 20161205 | 13.02% | 12.96% | 26.04% | 3.11% | 4.02% | 7.96% | No |
| 20170218 | 7.89% | 10.04% | 11.34% | 6.00% | 1.97% | 19.02% | No |
| 20170303 | 8.24% | 19.78% | 24.56% | 3.28% | 4.99% | 7.52% | No |
| 20170506 | 8.35% | 18.66% | 17.89% | 2.65% | 2.89% | 11.23% | No |
| 20170519 | 8.62% | 20.33% | 19.68% | 3.02% | 3.12% | 9.23% | No |

Preparation examples include but are not limited to the above.

## Claims

1. A pharmaceutical composition comprising zedoary turmeric oil and borneol, wherein the zedoary turmeric oil comprises germacrone, curdione, furanodiene, curcumol, β-elemene and curzerene, with no 2,4,6-pentamethylaniline, or the content of 2,4,6-pentamethylaniline is less than or equal to 100 ppm by weight.

2. The pharmaceutical composition according to claim 1, wherein the zedoary turmeric oil comprises 5.0-13.0 wt% of germacrone, 10.0 wt%-30.0 wt% of curdione, 10.0 wt%-26.0 wt% of furanodiene, 0.5 wt%-6.0 wt% of curcumol, 1.0 wt%-6.0 wt% of β-elemene, and 2.0 wt%-21.0 wt% of curzerene, with no 2,4,6-pentamethylaniline, or the content of 2,4,6-pentamethylaniline is less than or equal to 100 ppm by weight.

3. The pharmaceutical composition according to claim 1, wherein the zedoary turmeric oil comprises 6.0 wt%-12.0 wt% of germacrone, 13.0 wt%-26.0 wt% of curdione, 12.0 wt%-25.0 wt% of furanodiene, 0.8 wt%-5.0 wt% of curcumol, 2.0 wt%-5.0 wt% of β-elemene, 7.5 wt%-19.0 wt% of curzerene, with no 2,4,6-pentamethylaniline, or the content of 2,4,6-pentamethylaniline is less than or equal to 10 ppm by weight.

4. The pharmaceutical composition according to any one of claims 1 to 3, for use in the manufacture of a medicament for prevention and/or treatment of HPV infection, including infection or persistent infection, CINI, CINII, CINIII and cervical cancer caused by various subtypes of high-risk human papillomavirus (HR-HPV), and/or infection and genital condyloma acuminatum caused by various subtypes of low-risk human papillomavirus (LR-HPV).

5. The pharmaceutical composition according to claim 4, wherein the HR-HPV comprises HPV 16, 18, 30, 31, 33, 35, 39, 45, 51, 52, 53, 56, 58, 59, 66, 68, and 69; and the LR-HPV comprises HPV 6, 11, 42, 43, 44, 61 and CP8304.

6. The pharmaceutical composition according to any one of claims 1 to 3, formulated as a pharmaceutical preparation.

7. The pharmaceutical composition according to claim 6, wherein the pharmaceutical preparation is in a dosage form of an external preparation, an oral preparation, or an injection preparation.

8. The pharmaceutical composition according to claim 6, wherein the weight ratio of the zedoary turmeric oil to borneol in the pharmaceutical composition is 164: 150.

9. The pharmaceutical composition according to any one of claims 1 to 3, for use in a medicament for prevention and/or treatment of infection by various viruses, bacteria, fungi, chlamydiae, mycoplasmas, trichomonas; infection of eye, ear, nose and throat; vulvitis, various vaginitis, mixed infectious vaginitis, skin infection, mucosal infection, hemorrhoid infection, constipation, cervicitis, adnexitis, anti-oxidation.

10. The pharmaceutical composition according to claim 7, wherein the external preparation comprises the zedoary turmeric oil, borneol, and a transdermal absorption enhancer.

11. The pharmaceutical composition according to claim 10, wherein the transdermal absorption enhancer is one or more selected from laurocapram, dimethyl sulfoxide, tetradecanol, caprylic acid and oleic acid.
